(19) Europäisches Patentamt
European Patent Office
Office européen des brevets

(11) Veröffentlichungsnummer : **0 388 587 B1**

# (12) EUROPÄISCHE PATENTSCHRIFT

(45) Veröffentlichungstag der Patentschrift :
**11.11.92 Patentblatt 92/46**

(51) Int. Cl.$^5$ : **C07C 255/31,** C07C 255/34, C07C 255/07, C07C 253/30

(21) Anmeldenummer : **90101143.7**

(22) Anmeldetag : **20.01.90**

## (54) Verfahrensverbesserung bei der technisch angewandten Knoevenagelschen Synthese.

(30) Priorität : **18.03.89 DE 3908998**

(43) Veröffentlichungstag der Anmeldung :
**26.09.90 Patentblatt 90/39**

(45) Bekanntmachung des Hinweises auf die Patenterteilung :
**11.11.92 Patentblatt 92/46**

(84) Benannte Vertragsstaaten :
**AT BE CH DE FR GB IT LI NL**

(56) Entgegenhaltungen :
**EP-A- 0 149 054**
**FR-A- 1 342 572**

(56) Entgegenhaltungen :
**ORGANIKUM, 12. Auflage, 1973, Seiten 508-511, VEB Verlag, Berlin, DE**
**ORGANIC SYNTHESES, Band 31, 1951, Seiten 25-28, John Wiley & Sons, Inc., New York, US; A.C. COPE et al.:"Cyclohexylidenecyanoacetic acid and 1-cyclohexenylacetonitrile"**

(73) Patentinhaber : **HÜLS AKTIENGESELLSCHAFT**
**Patentabteilung / PB 15 - Postfach 13 20**
**W-4370 Marl 1 (DE)**

(72) Erfinder : **Kaufhold, Manfred, Dr.**
**Jasminweg 20**
**W-4370 Marl (DE)**
Erfinder : **Metz, Josef, Dr.**
**Am Erzschacht 31**
**W-4370 Marl (DE)**

EP 0 388 587 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Verfahrensverbesserung bei der technisch angewandten Knoevenagelschen Synthese bei der Kondensation von Cyanessigsäure mit Ketonen, wie z. B. Cyclohexanon und Cyclododecanon.

Diese Knoevenagelsche Synthese ist aus der Literatur seit langem bekannt. Eine allgemeine Zusammenfassung findet sich im Houben Weyl, Band 8, Seite 450. Hier wird als besonders vorteilhafte Arbeitsweise die Verwendung von schwachen Basen, wie Ammoniak, primäre und sekundäre Amine, vorgeschlagen. Es wird hier festgestellt, daß die Decarboxylierung der zunächst entstehenden Additionsprodukte durch basische Katalysatoren begünstigt wird.

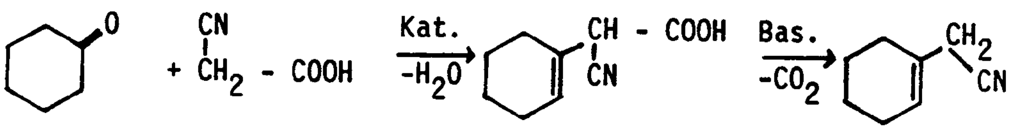


Als Beispiel wird dort diese Umsetzung von Cyclohexanon mit Cyanessigsäure beschrieben, wobei Ammonacetat in geringen Mengen als Katalysator eingesetzt wird. Die Reaktion erfolgt zweistufig. Das Reaktionsprodukt der ersten Stufe muß dann vorsichtig destilliert werden, weil dabei eine heftige Kohlendioxidabspaltung stattfindet.

In Org. Synth. Coll. IV, S. 234-236, ist diese Reaktion mit geringen Katalysatormengen und die Isolierung der Zwischenstufe, der Cyclohexenylcyanessigsäure, eingehender und exakter beschrieben. Auch hier wird gesagt, daß die Decarboxylierung vor oder bei der Destillation geschieht und dann "very rapidly".

Diese starke Gasentwicklung ist zwar bei Laborarbeiten kein Problem, sie verbietet aber eine technische Anwendung. Denn diese Gasentwicklung kann zum gefährlichen Zerplatzen von Apparaturen durch plötzlichen Überdruck führen. Diesen Druckstoß kann man sich zwar in Hochdruckapparaturen leisten, aber das bedeutet einen erheblich höheren Kostenaufwand.

Alle bekannten Verfahren zur technischen Durchführung der Knoevenagelschen Synthese haben den Nachteil, daß sie zu ungestüm und zu unregelmäßig verlaufen und deshalb aus Sicherheitsgründen einen hohen technischen Aufwand erfordern. Wünschenswert wäre ein Verfahren, bei dem sich die Abgasmenge regeln läßt und bei dem das Abgas in einem gleichmäßigen Strom ohne große Verzögerung anfällt, möglichst sofort nach Zusammengeben von Cyanessigsäure und Keton, d. h. die Zwischenstufe - im Falle des Cyclohexanon ist es die Cyclohexenylcyanessigsäure -, sollte sofort decarboxylieren.

Nach Organikum, 12. Auflage, VEB Deutscher Verlag der Wissenschaften, Berlin, 1973, Seite 508, ist eine Decarboxylierung des Kondensationsproduktes einstufig nach einer Verfahrensvariante von Knoevenagel zu erreichen, indem man Amine, wie z. B. Pyridin, in größeren Mengen als Lösemittel unter weiterem Zusatz von Piperidin verwendet. Durch den Einsatz dieser Amine in großen Mengen wird die Aufarbeitung des Reaktionsproduktes erheblich aufwendiger, und es entstehen Probleme der Abfallbeseitigung. Es wäre ein großer technischer Vorteil, wenn die schnelle Decarboxylierung der Kondensationsprodukte ohne basische Lösungsmittel möglich wäre. Des weiteren besteht ein großes Interesse an derartigen Verfahren, nach dem man bei geringem technischen Aufwand ohne Sicherheitsrisiko die Knoevenagelsche Synthese technisch durchführen kann.

Es war daher Aufgabe der Erfindung, nach einem Weg zu suchen, bei dem die Knoevenagelsche Synthese in einer Stufe ohne Probleme der Abfallbeseitigung und ohne Sicherheitsrisiko bei der Decarboxylierung erfolgt.

Gegenstand der Erfindung ist daher eine Verfahrensverbesserung bei der technisch angewandten Knoevenagelschen Synthese zur Umsetzung von Cyanessigsäure mit Ketonen in einer Stufe mit den dafür üblichen Katalysatoren, welches dadurch gekennzeichnet ist, daß man 1-10 Mol Essigsäure pro Mol Keton einsetzt und diese zusammen mit der Cyanessigsäure als Lösung zum Keton bei 10-160 °C gleichmäßig zugibt, wobei ein geregelter Abgasstrom entsteht.

Überraschenderweise wurde gefunden, daß die Decarboxylierung der Kondensationsprodukte von z. B. Cyclododecanon und Cyanessigsäure auch in saurer Lösung, in Gegenwart von Essigsäure, stattfinden kann, und daß bei Verwendung der größeren Mengen an Essigsäure die Abgasentwicklung gleichmäßig wird, sich regeln läßt und sofort nach Zusammengeben der Einsatzprodukte einsetzt. Dieser Effekt war nicht zu erwarten, denn wie oben ausgeführt, wird zur Decarboxylierung die Verwendung von basischen Verbindungen empfohlen. In der o. g. Arbeitsvorschrift des Organikums wird zwar gesagt, daß man auch geringe Mengen an Essigsäure zugeben kann, diese geringen Mengen können aber nur zur Salzbildung bzw. Stabilisierung des Katalysators dienen.

Mit der vom Organikum empfohlenen Essigsäuremenge, 0,2 Mol auf 1 Mol Keton, ist der erfindungsgemäße Effekt nicht zu erreichen. Die Essigsäuremengen müssen viel größer sein, und zwar bei 1 bis 10 Mol, vorzugsweise 1 bis 5 Mol pro Mol Keton, besonders bevorzugt bei 1,1 : 1, liegen. Auf die Verwendung eines Schleppmittels zum Auskreisen von Wasser kann man bei dem erfindungsgemäßen Verfahren verzichten.

Zweckmäßig löst man die feste Cyanessigsäure in Essigsäure in einem Gewichtsverhältnis von ca. 1 : 1, daraus resultiert ein bevorzugtes Molverhältnis Essigsäure zu Keton von 1,3 : 1. Diese bei Raumtemperatur flüssige Lösung dosiert man unter Messung des Abgases zu dem vorgelegten Keton und dem Katalysator. Die Herstellung dieser Lösung hat große Vorteile, da sich die Cyanessigsäure bei höheren Temperaturen zersetzt. Wenn man sie als Schmelze einsetzen will, kann es leicht zu Explosionen kommen, wenn wegen irgendwelcher technischer Mängel die Cyanessigsäure an einer Stelle der Apparatur überhitzt wird.

Die Reaktionstemperatur wird innerhalb eines Bereiches von 100 bis 160 °C je nach Reaktivität des Ketons so gewählt, daß bei der Zugabe der Lösung der Cyanessigsäure in Essigsäure Abgas entsteht. Der bevorzugte Temperaturbereich liegt z. B. beim Cyclododecanon bei 130 bis 140 °C und beim Cyclohexanon bei 100 bis 120 °C. Zu hohe Temperaturen führen in folge Zersetzung der Cyanessigsäure zu Ausbeuteverlusten.

Ansonsten gelten bei dem erfindungsgemäßen Verfahren die für die Knoevenagels-Synthese typischen Regeln. An Ketonen können aliphatische, cycloaliphatische, wie Cyclohexanon, Cycloheptanon, Cyclododecanon, Octanon-3, Octanon-2, Heptanon-2 usw. sowie Alkyl-Aryl-Ketone, wie Acetophenon, Benzophenon u. a., eingesetzt werden.

Die folgenden Beispiele sollen das erfindungsgemäße Verfahren näher erläutern.

<u>Vergleichsbeispiel 1</u>

Man benutzt eine Glasapparatur, die aus einem Vierhalskolben mit Rührer, Thermometer, Tropftrichter und Destillationskolonne mit aufgesetztem Wasserauskreiser und Rückflußkühler mit angeschlossener Gasuhr besteht.

Man setzt ein:

| | |
|---|---|
| 910 g (= 5 Mol) | Cyclododecanon |
| 300 g | Toluol |
| 20 g (= 0,259 Mol) | Ammonacetat |

Dieses Gemisch wird unter Rühren auf 132 °C erwärmt und zunächst nur 50 ml einer Lösung von insgesamt

| | |
|---|---|
| 389 g (= 4,3 Mol) | Cyanessigsäure und |
| 60,1 g (= 1,0 Mol) | Essigsäure |

zugetropft, wobei ein Essigsäure-Wassergemisch abdestilliert.

An der Gasuhr wird die in einer halben Stunde angefallene Menge an Abgas abgelesen, siehe Spalte 6 in der Tabelle 1. In der Spalte 5 ist die aus der Einsatzmenge errechnete Menge an Abgas eingesetzt, die bei spontaner Reaktion entstehen könnte. Die Spalte 7 zeigt die Summe der angefallenen Abgasmengen. Je geringer die Differenz ist zwischen den Werten in Spalte 5 und 7, umso geringer ist auch die Gefahr eines plötzlichen Druckanstiegs. Man sieht, daß die Differenz anfangs, z. B. nach der 1. und 2. Stunde, sehr groß ist, später z. B. nach der 8. und 10. Stunde etwas geringer wird. Geradezu dramatisch ist die Abgasmenge die entsteht, wenn man das Reaktionsgemisch abkühlt und wieder aufwärmt, was in der Technik manchmal erforderlich ist, siehe nach der 2. Stunde. Die hier gemessene Abgasmenge würde in großen technischen Geräten zu einem plötzlichen Druckanstieg, d. h. zu einer Explosion, führen.

Nach 16 Stunden ist die Reaktion beendet. Der Reaktionsaustrag wiegt 1 338 g. Nach gaschromatographischer Analyse enthält das Reaktionsprodukt 27,8 % Cyclododecanon und 69,0 % Cyclododecenylacetonitril. Hieraus errechnet sich ein Umsatz an Keton von 59,1 %. Die Ausbeute an Nitril, bezogen auf umgesetztes Keton, liegt bei 89,9 %.

<u>Beispiel 1</u>

Man benutzt die im Vergleichsbeispiel 1 beschriebene Apparatur und setzt ein:

| | |
|---|---|
| 910 g (= 5,0 Mol) | Cyclododecanon |
| 20 g (= 0,259 Mol) | Ammonacetat |

Das Gemisch wird unter Rühren auf 140 °C erwärmt und zunächst 50 ml einer Lösung von insgesamt

| | |
|---|---|
| 389 g (= 4,3 Mol) | Cyanessigsäure und |
| 389 g (= 6,48 Mol) | Essigsäure |

zugetropft, wobei ein Essigsäure-Wassergemisch abdestilliert.

Die Tabelle 2 zeigt den Verlauf der Reaktion und der Abgasentwicklung. Die Werte in der Spalte 5, das

sind die aus der Einsatzmenge berechneten Abgasmengen, zeigen im Vergleich mit den Zahlen der Spalte 6, der Summe der gemessenen Abgasmengen, nur überraschend geringe Abweichungen. Selbst nach Abstellen und wieder Anstellen, siehe nach der 8. Stunde, stellt sich wieder eine ähnliche Abgasmenge wie vorher ein. Die Abgas mengen untereinander sind auch sehr ähnlich, d. h. es entsteht schnell ein gleichmäßiger Abgasstrom, der für die technische Durchführung sehr wichtig ist.

Nach 16 Stunden ist die Reaktion beendet. Der Reaktionsaustrag wiegt 1 193 g. Nach gaschromatographischer Analyse enthält das Reaktionsprodukt 29,6 % Cyclododecanon und 49,4 % Cyclododecenylacetonitril. Hieraus errechnet sich ein Umsatz an Keton von 61,2 % und eine Ausbeute an Nitril, bezogen auf umgesetztes Keton, von 93,8 %.

Vergleichsbeispiel 2

Man benutzt die im Vergleichsbeispiel 1 beschriebene Apparatur und setzt ein:

| | |
|---|---|
| 490 g (= 5,0 Mol) | Cyclohexanon |
| 20 g (= 0,259 Mol) | Ammonacetat |

Das Gemisch wird unter Rühren auf 120 °C erwärmt und wie in der folgenden Tabelle angegeben, werden jeweils 30 ml einer Lösung, die aus

| | |
|---|---|
| 370 g (= 4,3 Mol) | Cyanessigsäure und |
| 60,1 g (= 1,0 Mol) | Essigsäure |

besteht, zugegeben.

Die Tabelle 3 zeigt den Verlauf der Reaktion und der Abgasentwicklung wie bei den vorherigen Beispielen: Man erkennt deutlich, daß nach 3 Stunden eine heftige Abgasentwicklung einsetzt, die doppelt so groß ist wie die vorherige Abgasmenge. Diese Problematik macht eine technische Nutzung unmöglich.

Die Ausbeute an Cyclohexenylacetonitril, berechnet aus dem gaschromatographisch ermittelten Gehalt im Reaktionsprodukt liegt bei 86 %.

Beispiel 2

Man benutzt die im Vergleichsbeispiel 1 beschriebene Apparatur und setzt ein:

| | |
|---|---|
| 490 g (= 5,0 Mol) | Cyclohexanon |
| 20 g (= 0,259 Mol) | Ammonacetat |
| 50 g | Cyclohexan |

Das Gemisch wird unter Rühren auf 100 °C erwärmt und wie in der folgenden Tabelle angegeben, werden jeweils 50 ml einer Lösung zugegeben, die aus

| | |
|---|---|
| 389 g (= 4,5 Mol) | Cyanessigsäure und |
| 389 g (= 6,48 Mol) | Essigsäure |

besteht.

Die weitere Durchführung erfolgt wie im Beispiel 1 beschrieben. Das Ergebnis zeigt Tabelle 4. Um bei der niedrigen Sumpftemperatur von 100 °C Destillatanfall zu bekommen, wurde, wie in der Tabelle angegeben, Cyclohexan zugegeben. Wie die Tabelle 4 zeigt, entsteht ein gleichmäßiger regelbarer Abgasstrom.

Die Ausbeute an Cyclohexenylacetonitril liegt bei 91 %.

Beispiel 3

Man benutzt die im Vergleichsbeispiel 1 beschriebene Apparatur und setzt die im Beispiel 2 (Keton, Cyclohexanon) aufgeführten Produkte ein, mit dem Unterschied, daß man anstelle von 6,48 Mol Essigsäure 25,0 Mol Essigsäure verwendet. Die Durchführung erfolgt wie im Beispiel 1 beschrieben.

Es entsteht ein gleichmäßiger regelbarer Abgasstrom. Die Ausbeute an Cyclohexenylacetonitril liegt bei 83 %.

Beispiel 4

Man benutzt die im Vergleichsbeispiel 1 beschriebene Apparatur und setzt ein:

| | |
|---|---|
| 600 g (= 5,0 Mol) | Acetophenon |
| 20 g (= 0,259 Mol) | Ammonacetat |
| 50 g | Cyclohexan |

Das Gemisch wird unter Rühren auf 120 °C erwärmt und, wie im Beispiel 1 gezeigt, ein Gemisch von

| | |
|---|---|
| 389 g (= 4,5 Mol) | Cyanessigsäure und |

389 g (= 6,48 Mol) Essigsäure

portionsweise zugegeben. Es entsteht ein gleichmäßiger regelbarer Gasstrom. Wenn der Gasstrom nachläßt, wird die Temperatur auf 130 und später auf 140 °C erhöht. Nach 17 Stunden ist die Reaktion beendet.

Die destillative Aufarbeitung liefert das 3-Methylzimtsäurenitril in 75 %iger Ausbeute.

## Beispiel 5

Man benutzt die im Vergleichsbeispiel 1 beschriebene Apparatur und setzt ein:

| | |
|---|---|
| 640 g (= 5,0 Mol) | Octanon-3 |
| 20 g (= 0,259 Mol) | Ammonacetat |
| 50 g | Cyclohexan |

Das Gemisch wird unter Rühren auf 130 °C erwärmt und, wie im Beispiel 1 gezeigt, ein Gemisch von

| | |
|---|---|
| 389 g (= 4,5 Mol) | Cyanessigsäure und |
| 389 g (= 6,48 Mol) | Essigsäure |

portionsweise zugegeben. Es entsteht ein gleichmäßiger regelbarer Gasstrom. Wenn der Gasstrom nachläßt, wird die Temperatur auf 140 °C erhöht. Nach 20 Stunden ist die Reaktion beendet.

Die destillative Aufarbeitung liefert das β-Ethyl/β-Amyl-acrylnitril im Gemisch mit Doppelbindungsisomeren in 71 %iger Ausbeute.

## Beispiel 6

Man benutzt die im Vergleichsbeispiel 1 beschriebene Apparatur und setzt die im Beispiel 2 (Keton, Cyclohexanon) aufgeführten Produkte ein, mit dem Unterschied, daß man anstelle von 0,259 Mol Ammonacetat nun 0,3 Mol Piperidin einsetzt. Die Durchführung erfolgt wie im Beispiel 1 beschrieben.

Es entsteht ein gleichmäßiger regelbarer Abgasstrom. Die Ausbeute an Cyclohexenylacetonitril liegt bei 86 %.

<u>Tabelle 1</u> (Vergleichsbeispiel 1)

| Spalte | 1 | 2 | 3 | 4 | 5 | 6 | 7 |
|---|---|---|---|---|---|---|---|
| | Dauer | Sumpftemp. | Zulauf | | | Abgas | |
| | in h | °C | ml 1/2 h | gesamt | soll | l 1/2 h | ist |
| | Beg. | 120 | - | - | - | - | - |
| | 1/2 | 132 | 50 | 50 | 15,1 | 0,8 | 0,8 |
| | 1 | " | 50 | 100 | 30,2 | 2,4 | 3,2 |
| | 1/2 | 134 | 50 | 150 | 45,3 | 4,6 | 7,8 |
| | 2 | " | - | 150 | 45,3 | 7,7 | 15,5 |
| | Beg. | 134 | - | 150 | 45,3 | 3,0 | 18,5 |
| | 1/2 | " | - | 150 | 45,3 | 19,5 | 38,0 |
| | 3 | 136 | 30 | 180 | 54,5 | 3,0 | 41,0 |
| | 1/2 | " | - | 180 | 54,5 | 3,6 | 44,6 |
| | 4 | " | - | 180 | 54,5 | 0,5 | 45,1 |
| | 1/2 | " | 30 | 210 | 63,6 | 4,3 | 49,3 |
| | 5 | " | - | 210 | 63,6 | 2,8 | 52,1 |
| | 1/2 | " | 30 | 240 | 72,6 | 0,1 | 52,2 |
| | 6 | " | - | 240 | 72,6 | 3,5 | 55,7 |
| | 1/2 | " | - | 240 | 72,6 | 4,2 | 59,9 |
| | 7 | " | - | 240 | 72,6 | 0,2 | 60,1 |
| | 1/2 | " | 30 | 270 | 81,7 | 4,0 | 64,1 |
| | 8 | 137 | - | 270 | 81,7 | 2,0 | 66,1 |
| | 1/2 | " | 30 | 300 | 90,8 | 2,8 | 68,9 |
| | 9 | " | - | 300 | 90,8 | 3,6 | 72,5 |
| | Beg. | 138 | - | 300 | 90,8 | 1,7 | 74,2 |
| | 1/2 | 140 | - | 300 | 90,8 | 0,4 | 74,6 |
| | 10 | " | 30 | 330 | 99,9 | 1,5 | 76,1 |
| | 1/2 | " | - | 330 | 99,9 | 2,0 | 78,1 |
| | 11 | " | 30 | 360 | 109,0 | 1,1 | 79,2 |
| | 1/2 | " | 10 | 370 | 112,0 | 0,9 | 80,1 |
| | 12 | " | | | 112,0 | 0,9 | 81,0 |
| | 1/2 | " | | | 112,0 | 0,6 | 81,6 |
| | 13 | " | | | 112,0 | 0,8 | 82,4 |
| | 1/2 | " | | | 112,0 | 0,7 | 83,1 |

Tabelle 1 (Vergleichsbeispiel 1) - Fortsetzung -

| Spalte | 1 | 2 | 3 | 4 | 5 | 6 | 7 |
|---|---|---|---|---|---|---|---|
| | Dauer | Sumpftemp. | Zulauf | | | Abgas | |
| | in h | °C | ml 1/2 h | gesamt | soll | l 1/2 h | ist |
| | 14 | " | | | 112,0 | 0,7 | 83,8 |
| | 1/2 | " | | | 112,0 | 0,6 | 84,4 |
| | 15 | " | | | 112,0 | 0,5 | 84,9 |
| | 1/2 | " | | | 112,0 | 0,3 | 85,1 |
| | 16 | " | | | 112,0 | 0,2 | 85,3 |

<u>Tabelle 2</u> (Beispiel 1)

| Spalte | 1 | 2 | 3 | 4 | 5 | 6 | 7 |
|---|---|---|---|---|---|---|---|
| | Dauer in h | Sumpftemp. °C | Zulauf ml 1/2 h | gesamt | soll | Abgas ist | l 1/2 h |
| | Beg. | 140 | - | - | - | - | - |
| | 1/2 | " | 50 | 50 | 7,4 | 6,3 | 6,3 |
| | 1 | " | 50 | 100 | 14,7 | 14,0 | 7,7 |
| | 1/2 | " | 50 | 150 | 22,1 | 21,6 | 7,6 |
| | 2 | " | 50 | 200 | 29,5 | 29,6 | 8,0 |
| | 1/2 | " | 50 | 250 | 36,8 | 36,8 | 7,2 |
| | 3 | " | 50 | 300 | 44,2 | 43,9 | 7,1 |
| | 1/2 | " | 50 | 350 | 51,6 | 51,3 | 7,4 |
| | 4 | " | 50 | 400 | 59,0 | 56,1 | 4,8 |
| | 1/2 | " | 50 | 450 | 66,3 | 58,8 | 2,7 |
| | 5 | " | - | 450 | 66,3 | 62,0 | 3,2 |
| | 1/2 | " | - | 450 | 66,3 | 64,0 | 2,0 |
| | 6 | " | 50 | 500 | 73,7 | 65,2 | 1,2 |
| | 1/2 | " | - | 500 | 73,7 | 66,2 | 1,0 |
| | 7 | " | - | 500 | 73,7 | 67,3 | 1,1 |
| | 1/2 | " | 50 | 550 | 81,1 | 67,9 | 0,6 |
| | 8 | " | - | 550 | 81,1 | 68,9 | 1,0 |
| | Beg. | 132 | - | 550 | 81,1 | 68,9 | - |
| | 1/2 | 140 | - | 550 | 81,1 | 69,2 | 0,3 |
| | 9 | " | - | 550 | 81,1 | 70,2 | 1,0 |
| | 1/2 | " | - | 550 | 81,1 | 71,3 | 1,1 |
| | 10 | " | - | 550 | 81,1 | 72,3 | 1,0 |
| | 1/2 | " | - | 550 | 81,1 | 73,3 | 1,0 |
| | 11 | " | - | 550 | 81,1 | 74,2 | 0,9 |
| | 1/2 | " | - | 550 | 81,1 | 74,6 | 0,4 |
| | 12 | " | 50 | 600 | 88,4 | 75,5 | 0,9 |
| | 1/2 | " | - | 600 | 88,4 | 76,0 | 0,5 |
| | 13 | " | 50 | 650 | 95,8 | 77,0 | 1,0 |
| | 1/2 | " | 20 | 670 | 98,7 | 78,2 | 1,2 |

Tabelle 2 (Beispiel 1) - Fortsetzung -

| Spalte | 1 | 2 | 3 | 4 | 5 | 6 | 7 |
|---|---|---|---|---|---|---|---|
| | Dauer in h | Sumpftemp. °C | Zulauf ml 1/2 h | gesamt | soll | Abgas ist | l 1/2 h |
| | 14 | " | | | 98,7 | 79,4 | 1,2 |
| | 1/2 | " | | | 98,7 | 80,6 | 1,2 |
| | 15 | " | | | 98,7 | 81,7 | 1,1 |
| | 1/2 | " | | | 98,7 | 83,0 | 1,3 |
| | 16 | " | | | 98,7 | 84,0 | 1,4 |

Tabelle 3 (Vergleichsbeispiel 2)

| Dauer | Sumpftemperatur °C | Zulauf ml/h | gesamt | Abgas soll | ist | l / 1/2 h |
|---|---|---|---|---|---|---|
| Beg. | 120 | - | - | - | - | - |
| 1/2 | " | 30 | 30 | 8,6 | 7,1 | 7,1 |
| 1 | " | 30 | 60 | 17,2 | 14,5 | 7,4 |
| 1/2 | " | 30 | 90 | 25,6 | 22,7 | 8,2 |
| 2 | " | 30 | 120 | 34,3 | 31,0 | 8,3 |
| 1/2 | " | 30 | 150 | 42,9 | 39,0 | 8,0 |
| 3 | " | 30 | 180 | 51,5 | 57,4 | 18,4 |
| 1/2 | " | - | 180 | 51,5 | 58,0 | 0,6 |
| Beg. | " | - | 180 | 51,5 | 58,0 | - |
| 4 | " | 30 | 210 | 60,1 | 64,5 | 6,5 |
| 1/2 | " | 30 | 240 | 68,7 | 71,0 | 6,5 |
| 5 | " | 30 | 270 | 77,3 | 78,3 | 6,3 |
| 1/2 | " | 30 | 300 | 85,3 | 84,5 | 6,2 |
| 6 | 118 | - | 300 | 85,3 | 86,1 | 1,6 |
| 1/2 | 116 | 30 | 330 | 94,4 | 91,2 | 5,1 |
| 7 | 114 | - | 330 | 94,4 | 92,4 | 1,2 |
| 1/2 | " | 30 | 360 | 103,0 | 96,2 | 3,8 |
| 8 | " | | | 103,0 | 98,5 | 2,3 |
| 1/2 | 112 | | | 103,0 | 99,1 | 0,6 |
| 9 | " | | | 103,0 | 99,2 | 0,1 |
| 1/2 | " | | | 103,0 | 99,2 | 0,0 |

Tabelle 4 (Beispiel 3)

| Dauer | Sumpftemp. °C | Zulauf ml/1/2 h | Zulauf gesamt | Abgas soll | Abgas ist | Abgas 1/1/2 h | Cyclohexan-zugabe |
|---|---|---|---|---|---|---|---|
| Beg. | 100 | - | - | - | - | - | |
| 1/2 | " | 50 | 50 | 8,0 | 5,9 | 5,9 | |
| 1 | " | 50 | 100 | 16,0 | 13,2 | 6,3 | 25 g |
| 1/2 | " | 50 | 150 | 24,0 | 20,7 | 7,5 | |
| 2 | " | 50 | 200 | 32,0 | 27,9 | 7,2 | |
| 1/2 | " | 50 | 250 | 40,0 | 33,7 | 5,8 | |
| 3 | " | 50 | 300 | 48,0 | 38,4 | 4,7 | |
| 1/2 | " | - | 300 | 48,0 | 41,7 | 3,3 | 25 g |
| 4 | " | - | 300 | 48,0 | 42,3 | 0,6 | |
| 1/2 | " | 50 | 350 | 56,0 | 45,8 | 3,5 | |
| 5 | " | - | 350 | 56,0 | 47,7 | 1,9 | |
| 1/2 | " | - | 350 | 56,0 | 48,5 | 0,8 | 25 g |
| 6 | " | 50 | 400 | 64,0 | 50,9 | 2,4 | |
| Beg. | " | - | 400 | 64,0 | - | - | |
| 1/2 | " | - | 400 | 64,0 | 54,9 | 4,4 | |
| 7 | " | 50 | 450 | 72,0 | 57,8 | 2,9 | |
| 1/2 | " | 50 | 500 | 80,0 | 59,5 | 1,7 | |
| 8 | " | - | 500 | 80,0 | 62,9 | 3,4 | |
| 1/2 | " | - | 500 | 80,0 | 66,2 | 3,3 | |
| 9 | " | 50 | 550 | 88,0 | 67,9 | 1,7 | |
| 1/2 | " | - | 550 | 88,0 | 69,9 | 2,0 | |
| 10 | " | - | 550 | 88,0 | 71,8 | 1,9 | |
| 1/2 | " | - | 550 | 88,0 | 73,1 | 1,3 | |
| 11 | " | 50 | 600 | 96,0 | 73,9 | 0,8 | 25 g |
| 1/2 | " | - | 600 | 96,0 | 74,9 | 1,0 | |
| 12 | " | - | 600 | 96,0 | 76,6 | 1,7 | |
| 1/2 | " | - | 600 | 96,0 | 77,1 | 1,1 | |
| 13 | " | 50 | 650 | 104,0 | 78,3 | 0,6 | |
| 1/2 | " | - | 650 | 104,0 | 79,1 | 0,8 | |
| 14 | " | 30 | 680 | 108,5 | 79,9 | 0,8 | |

Tabelle 4 (Beispiel 3) - Fortsetzung -

| Dauer | Sumpftemp. | Zulauf | | | Abgas | | Cyclohexan- |
|---|---|---|---|---|---|---|---|
| | °C | ml/1/2 h | gesamt | soll | ist | 1/1/2 h | zugabe |
| Beg. | " | | | 108,5 | 80,8 | 0,9 | |
| 1/2 | " | | | 108,5 | 81,2 | 0,4 | |
| 15 | " | | | 108,5 | 82,0 | 0,8 | |
| 1/2 | " | | | 108,5 | 82,8 | 0,8 | |
| 16 | " | | | 108,5 | 83,7 | 0,9 | |
| 1/2 | " | | | 108,5 | 84,1 | 0,4 | |
| 17 | " | | | 108,5 | 84,5 | 0,4 | |
| 1/2 | " | | | 108,5 | 84,9 | 0,4 | |

## Patentansprüche

1. Verfahrensverbesserung bei der technisch angewandten Knoevenagelschen Synthese zur Umsetzung von Cyanessigsäure mit Ketonen in einer Stufe, mit den dafür üblichen Katalysatoren,
   dadurch gekennzeichnet,
   daß man 1 - 10 Mol Essigsäure pro Mol Keton einsetzt und diese zusammen mit der Cyanessigsäure als Lösung zum Keton bei 100 - 160 °C gleichmäßig zugibt, wobei ein geregelter Abgasstrom entsteht.

2. Verfahren nach Anspruch 1,
   dadurch gekennzeichnet,
   daß man bevorzugt 1 - 5 Mol Essigsäure pro Mol Keton einsetzt.

3. Verfahren nach Anspruch 1,
   dadurch gekennzeichnet,
   daß man besonders bevorzugt 1,3 Mol Essigsäure pro Mol Keton einsetzt.

## Claims

1. A process improvement in the industrially applied Knoevenagel synthesis for the single-stage reaction of cyano acetic acid with ketones, using the catalysts customary for the purpose, characterized in that 1 - 10 mole of acetic acid is used per mole of ketone and the acetic acid is steadily added together with the cyano acetic acid in the form of a solution to the ketone at 100 - 160°C, the result being a controlled waste gas flow.

2. A process according to claim 1, characterized in that preferably 1 - 5 mole of acetic acid is used per mole of ketone.

3. A process according to claim 1, characterized in that particularly preferably 1.3 mole of acetic acid is used per mole of ketone.

## Revendications

1. Amélioration du procédé dans la synthèse selon Knoevenagel appliquée industriellement de mise en réaction de l'acide cyanacétique avec des cétones en une étape avec les catalyseurs habituels pour cela, caractérisé en ce que l'on met en oeuvre 1 à 10 mol d'acide acétique par mol de cétone et que l'on ajoute celui-ci avec l'acide cyanacétique comme solution, pour la Cétone à 100-160°C d'une manière régulière, grâce à quoi un courant de gaz d'échappement contrôlé se produit.

2. Procédé selon la revendication 1, caractérisé en ce que l'on met en oeuvre de préférence 1 à 5 mol d'acide acétique par mol de cétone.

3. Procédé selon la revendication 1, caractérisé en ce que l'on met en oeuvre d'une manière particulièrement préférée 1,3 mol d'acide acétique.